(19) Europäisches Patentamt
European Patent Office
Office européen des brevets

(11) Veröffentlichungsnummer: **0 342 565 B1**

(12) **EUROPÄISCHE PATENTSCHRIFT**

(45) Veröffentlichungstag der Patentschrift: **11.08.93**

(51) Int. Cl.$^5$: **A61K 31/425**

(21) Anmeldenummer: **89108663.9**

(22) Anmeldetag: **13.05.89**

# (54) Verwendung des Thiazol-Derivates Tiprotimod zur Herstellung eines Mittels zur Therapie von Virusinfektionen.

(30) Priorität: **16.05.88 DE 3816603**

(43) Veröffentlichungstag der Anmeldung:
**23.11.89 Patentblatt 89/47**

(45) Bekanntmachung des Hinweises auf die Patenterteilung:
**11.08.93 Patentblatt 93/32**

(84) Benannte Vertragsstaaten:
**AT BE CH DE ES FR GB GR IT LI LU NL SE**

(56) Entgegenhaltungen:
**EP-A- 0 194 572**
**DE-A- 2 006 080**
**DE-A- 2 700 951**

**DRUGS OF TODAY, Band 26, Nr. 4, 1990, Seiten 269-277; G.H. WERNER: "Immunomodulators as antiviral agents"**

(73) Patentinhaber: **BEHRINGWERKE Aktiengesellschaft**
**Postfach 1140**
**W-3550 Marburg 1(DE)**

(72) Erfinder: **Schorlemmer, Hans Ulrich, Dr.**
**Am Kirschenwald 2**
**W-3550 Marburg(DE)**
Erfinder: **Sedlacek, Hans Harald, Dr.**
**Sonnenhang 3**
**W-3550 Marburg(DE)**
Erfinder: **Hilfenhaus, Joachim, Dr.**
**Auf'm Gebrande 24**
**W-3550 Marburg(DE)**

(74) Vertreter: **Fischer, Hans-Jürgen, Dr. et al**
**Hoechst AG, Zentrale Patentabteilung, Gebäude F 821**
**W-6230 Frankturt am Main 80 (DE)**

EP 0 342 565 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.6/3.3.1)

## Beschreibung

Die Erfindung betrifft die Verwendung des Thiazol-Derivates Tiprotimod zur Herstellung eines Arzneimittels gegen Virusinfektionen bei Mensch und Tier.

Verfahren zur Herstellung von Tiprotimod sowie seine immunmodulatorische Wirkung sind in der deutschen Offenlegungsschrift DE 35 08 665 A1 beschrieben und für Tiprotimod die Struktur

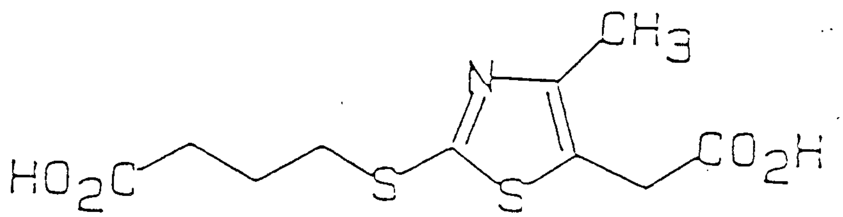

und der Name 2-(3-Carboxy-1-propylthio)-4-methyl-1,3-thiazol-5-yl-acetic acid angegeben.

Immunologische Untersuchungen ergaben, daß Zusammenhänge zwischen der natürlichen oder durch äußere Faktoren provozierten Abnahme der immunologischen Aktivität und der Zunahme der Infektions- oder Tumorgefahr bestehen. Das funktionelle Zusammenwirken aller Komponenten des Immunsystems, der humoralen und zellulären Immunität, ist für die Abwehrmechanismen des lebenden Organismus und damit für die Elimination von Fremdkörpern und Krankheitserregern, vornehmlich Mikroorganismen oder neoplastischen Zellen von großer Bedeutung.

Es wurde nun überraschend gefunden, daß die Substanz Tiprotimod neben ihrer Wirkung als Immunstimulator bei warmblütigen Säugern in einer Dosis, welche optimal zwischen 0,5 und 10 mg/kg Körpergewicht liegt und welche parenteral ein- bis mehrfach verabreicht wird, einen therapeutischen Einfluß auf virale Infektionserkrankungen besitzt, ohne dabei toxische Nebenwirkungen zu zeigen. Damit ist Tiprotimod für die Behandlung solcher Krankheiten geeignet. Im Tierversuch ist es beispielsweise möglich, eine tödlich verlaufende Herpes simplex Infektion bei Mäusen durch therapeutische Gabe von Tiprotimod so günstig zu beeinflussen, daß am Ende des Beobachtungszeitrames (25 Tage) eine Überlebensrate von 40 % resultiert. In der Kontrollgruppe überlebt im gleichen Beobachtungszeitraum kein Tier.

Aus DE-A-20 06 080 ist eine antivirale Wirkung bestimmter Thiazolidinderivate und aus DE-A-27 00 951 eine solche bestimmter Thiazolderivate bekannt. Von diesen Verbindungen ist Tiprotimod strukturell beträchtlich verschieden.

Gegenstand der Erfindung ist demzufolge die Verwendung von Tiprotimod zur Herstellung eines Arzneimittels für die Therapie von viralen Infektionserkrankungen, vorzugsweise von solchen verursacht durch Herpes simplex- oder EMC (Encephalomyocarditis)-Viren, entsprechend den Patentansprüchen.

Die wirksame therapeutische Menge liegt im Bereich von 0.1-100 mg/kg, vorzugsweise 0.5-10 mg/kg Körpergewicht bei parenteraler Verabreichung.

Der Wirkstoff kann alleine gegeben oder auch mit anderen Arzneimitteln kombiniert werden, die Infektionen günstig beeinflussen. Der Wirkstoff kann erfindungsgemäß sowohl parenteral als auch oral verabreicht werden. Für die parenterale, speziell intravenöse Verabreichung kommen Lösungen oder Suspensionen des Wirkstoffes in einem bekannten pharmazeutisch verträglichen Vektor in Betracht.

Zur Herstellung wäßriger Lösungen wird der Wirkstoff vorzugsweise in Form wasserlöslicher, physiologisch verträglicher Salze eingesetzt. Die Zubereitungen können die üblichen Hilfs- und Trägerstoffe enthalten. Als solche kommen beispielsweise Füllstoffe, Emulgatoren, Gleit- und Pufferstoffe und geschmackskorrigierende Agenzien in Frage.

Die entsprechend der Erfindung verwendete Verbindung zeigt in den Konzentrationsbereichen, in denen sie wirksam ist, keine Toxizität und führt nicht zur lokalen Granulombildung.

Im folgenden wird die Wirkung der Substanz in Standardtestmethoden beispielhalt erläutert. Die herangezogenen verschiedenen Testmodelle sind bekanntermaßen für die Beurteilung der Wirkungsqualität von Therapeutika gegen Virusinfektionen bei Mensch und Tier besonders gut geeignet.

Beispiel 1

Therapeutische Behandlung einer Herpes simplex HSV-1 Virusinfektion

Zur therapeutischen Behandlung einer Herpes simplex HSV-1 Virusinfektion wurden haarlose Mäuse (10/Gruppe) am Tag 0 intradermal mit 0.1 ml pro Maus Virussuspension (Stamm. "Wal"; 1000 $LD_{50}$) infiziert. Die Testsubstanz wurde den Tieren entweder einmal oder in zwei aufeinanderfolgenden Tagesgaben in Konzentrationen von 0.8, 2.4 und 7.2 mg/kg Körpergewicht intraperitoneal verabreicht (siehe Tabelle 1). Die Tiere wurden täglich über einen Zeitraum von 25 Tagen auf Überleben kontrolliert und die Überlebensrate bestimmt. Wie aus der Tabelle 2 zu ersehen ist, sterben alle Tiere in der unbehandelten Kontrollgruppe. Eine zweimalige Injektion der Testsubstanz (7.2 mg/kg) an den Tagen 1 und 2 nach Infektion führt zu einem Schutz (40 %) vor der Virusinfektion. Die Prüfsubstanz reduziert selbst bei einmaliger Gabe am Tag 2 nach der Infection deutlich die Absterberate (40 %) und beeinflußt damit die Erkrankung therapeutisch.

Beispiel 2

Beeinflussung der EMC-Virusmyokarditis der NMRI Maus

Hier wurden NMRI Mäuse im Alter von 6 Wochen zunächst mit dem kardiotropen EMC-Virus (5.5 PFU = plaque forming units) intraperitoneal infiziert und anschließend in der Akutphase der Erkrankung bis zum Tag 10 jeden 2. Tag mit 1 mg/kg intraperitoneal ab dem 1. Tag nach der Infektion therapiert. Um den Effekt auf die Frühphase der viralen Herzerkrankung zu untersuchen, wurden Lethalität, Gewichtsverlauf, Hämodynamik und histologische Parameter der Myokarditis bei den Mäusen erfaßt. Aus den vorliegenden Ergebnissen ist zu ersehen, daß die Gabe von Tiprotimod (1 mg/kg i.p. jeden 2. Tag) zu einer Verbesserung histologischer Parameter bei Mäusen 10 Tage nach einer EMC Virusinfektion führt. In Übereinstimmung hiermit steht die Beeinflussung der Lethalität, hämodynamischer Parameter und der günstige Effekt auf Lähmungserscheinungen in der Akutphase der Erkrankung.

Tiprotimod ist also in in vivo-Testmodellen, die für die Beurteilung von antiviral wirksamen Therapeutika herangezogen werden können, imstande, die Überlebensrate der infizierten Tiere deutlich zu erhöhen und die Symptome zu lindern. Tiprotimod kann also als Therapeutikum bei Virusinfektionen angewendet werden.

Tabelle 1

Behandlung einer HSV1 Infektion bei haarlosen Mäusen mit Tiprotimod - Behandlungsschema

Beobachtungszeitraum (Tag 0 – Tag 25)

| Gruppe | Tag -1<br>Tiprotimod<br>0.8, 2.4, 7.2 mg/kg<br>1 x i.p. | Tag 0<br>HSV1-Infektion(id)<br>0.1 ml/Maus;<br>33 902 1:20 | Tag 1<br>Tiprotimod<br>0.8, 2.4, 7.2 mg/kg<br>1 x i.p. | Tag 2<br>Tiprotimod<br>0.8, 2.4, 7.2 mg/kg<br>1 x i.p. | Tag 25 |
|---|---|---|---|---|---|
| 1. | | | | | |
| 2. | ↓ | ↓ | | | |
| 3. | | ↓ | ↓ | ↓ | |
| 4. | | ↓ | | ↓ | |

Tabelle 2

| Behandlung einer HSV-1 Infektion bei haarlosen Mäusen mit Tiprotimod - Ergebnisse | | | | | |
|---|---|---|---|---|---|
| Gruppe | HSV-1 Infektion | Prophylaxe/Therapie mit Tiprotimod überlebende Tiere | | | |
| | | 0 | 0.8 | 2.4 | 7.2 mg/kg |
| 1. | + | 0/10 | | | |
| 2. | + | | 0/10 | 1/10 | 1/10 |
| 3. | + | | 3/10 | 2/10 | 4/10 |
| 4. | + | | 1/10 | 3/10 | 4/10 |

Tabelle 3

| Therapeutische Beeinflussung der viral (EMC Virus) induzierten Myokarditis bei NMRI Mäusen in der Akutphase bis zum 10. Tag der Erkrankung | | |
|---|---|---|
| Meßparameter | unbehandelte, EMC-infizierte Kontrollgruppe | Therapiegruppe 1 mg/kg Tiprotimod/i.p. jeden 2. Tag |
| Gewicht: | | |
| 1. Tag | 24.9 ± 2.4 g | 26.1 ± 1.8 g |
| 10. Tag | 27.7 ± 6.5 g | 32.0 ± 3.4 g |
| systolischer Druck des linken Herzventrikels: | 60.0 ± 9.0 mm Hg | 81.0 ± 15.0 15 mm Hg |
| Histologie des Herzens: | | |
| Lymphozytäres Infiltrat: | 11/14 (78.6 %) | 2/20 (10 %) |
| Fibrose: | 0 | 0 |
| Fokale Nekrosen: | 10/14 (71.4 %) | 3/20 (15 %) |
| Verkalkung: | 2/14 (14.3 %) | 0 |
| Lähmung: | 8 Tiere | 0 |
| Todesfälle: | 6 Tiere | 0 |

**Patentansprüche**

**Patentansprüche für folgende Vertragsstaaten : AT, BE, CH, DE, FR, GB, IT, LI, LU, NL, SE**

1. Verwendung von Tiprotimod zur Herstellung eines Arzneimittels für die Therapie von viralen Infektionserkrankungen.

2. Verwendung nach Anspruch 1, dadurch gekennzeichnet, daß das Virus Herpes simplex- oder EMC-Virus ist.

3. Verwendung von Tiprotimod nach Anspruch 1, dadurch gekennzeichnet, daß das Arzneimittel Tiprotimod in einer Menge von 0,1-100 mg/kg, vorzugsweise 0.5-10 mg/kg, Körpergewicht enthält.

**Patentansprüche für folgende Vertragsstaaten : ES, GR**

1. Verfahren zur Herstellung eines Arzneimittels für die Therapie von viralen Infektionserkrankungen, dadurch gekennzeichnet, daß Tiprotimod in eine für eine Darreichung geeignete Form gebracht wird.

2. Verfahren nach Anspruch 1, dadurch gekennzeichnet, daß das Arzneimittel Tiprotimod in einer Menge von 0,1-100 mg/kg, vorzugsweise 0.5-10 mg/kg, Körpergewicht enthält.

3. Verfahren nach Anspruch 1, dadurch gekennzeichnet, daß ein Arzneimittel zur Behandlung von Infektionskrankheiten hergestellt wird, die durch Herpes simplex- oder EMC-Virus verursacht sind.

**4.** Verfahren nach Anspruch 1, dadurch gekennzeichnet, daß Tiprotimod zusammen mit Hilfs- und oder Zusatzstoffen zu einem Arzneimittel verarbeitet wird.

**Claims**

**Claims for the following Contracting States : AT, BE, CH, DE, FR, GB, IT, LI, LU, NL, SE**

**1.** The use of tiprotimod for the preparation of a pharmaceutical for the therapy of viral infectious diseases.

**2.** The use as claimed in claim 1, wherein the virus is herpes simplex or EMC virus.

**3.** The use of tiprotimod as claimed in claim 1, wherein the pharmaceutical contains tiprotimod in an amount of 0.1-100 mg/kg, preferably 0.5-10 mg/kg, of body weight.

**Claims for the following Contracting States : ES, GR**

**1.** A process for the preparation of a pharmaceutical for the therapy of viral infectious diseases, which comprises converting tiprotimod into a form suitable for an administration.

**2.** The process as claimed in claim 1, wherein the pharmaceutical contains tiprotimod in an amount of 0.1-100 mg/kg, preferably 0.5-10 mg/kg, of body weight.

**3.** The process as claimed in claim 1, wherein a pharmaceutical for the treatment of infections diseases caused by herpes simplex or EMC virus is prepared.

**4.** The process as claimed in claim 1, wherein tiprotimod is processed together with auxiliaries or additives to give a pharmaceutical.

**Revendications**

**Revendications pour les Etats contractants suivants : AT, BE, CH, DE, FR, GB, IT, LI, LU, NL, SE**

**1.** Utilisation du tiprotimod pour la fabrication d'un médicament pour le traitement de maladies infectieuses virales.

**2.** Utilisation selon la revendication 1, caractérisée en ce que le virus est le virus Herpes simplex ou EMC.

**3.** Utilisation du tiprotimod selon la revendication 1, caractérisée en ce que le médicament contient du tiprotimod en une quantité de 0,1-100 mg/kg, de préférence de 0,5-10 mg/kg de poids corporel.

**Revendications pour les Etats contractants suivants : ES, GR**

**1.** Procédé pour la fabrication d'un médicament pour le traitement de maladies infectieuses virales, caractérisé en ce que l'on met du tiprotimod sous une forme pharmaceutique appropriée.

**2.** Procédé selon la revendication 1, caractérisé en ce que le médicament contient du tiprotimod en une quantité de 0,1-100 mg/kg, de préférence de 0,5-10 mg/kg de poids corporel.

**3.** Procédé selon la revendication 1, caractérisé en ce que l'on fabrique un médicament pour le traitement de maladies infectieuses qui sont provoquées par le virus Herpes simplex ou EMC.

**4.** Procédé selon la revendication 1, caractérisé en ce que l'on transforme en médicament du tiprotimod avec des adjuvants et/ou additifs.